# FASCICULE DE BREVET EUROPEEN

(11) **EP 0 701 433 B1**
(45) Date de publication et mention de la délivrance du brevet: **02.05.1997**
(21) Numéro de dépôt: 94932153.3
(22) Date de dépôt: 31.05.1994
(51) Int. Cl.: A61K 7/50

(54) **COMPOSITIONS COSMETIQUES CONTENANT AU MOINS UN TENSIO-ACTIF ANIONIQUE DU TYPE ALKYLGALACTOSIDE URONATE ET AU MOINS UN TENSIO-ACTIF DU TYPE ALKYLPOLYGLYCOSIDE ET/OU POLYGLYCEROLE**
KOSMETISCHEMITTEL ENTHALTENDE MINDESTENS EINEN ANIONISCHEN TENSIDE VOM TYP ALKYLGALACTOSIDE URONAT UND MINDESTENS EINEN ANIONISCHEN TENSIDE VOM TYP ALKYLPOLYGLUCOSID UND/ODER POLYGYCEROLIERT
COSMETIC COMPOUNDS CONTAINING AT LEAST ONE ANIONIC ALKYLGALACTOSIDE URONATE TYPE SURFACE-ACTIVE AGENT AND AT LEAST ONE ALKYLPOLYGLYCOSIDE AND/OR POLYGLYCEROL TYPE SURFACE-ACTIVE AGENT

(30) Priorité: 01.06.1993 FR 9306529
(43) Date de publication de la demande: 20.03.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400630
(87) Numéro de publication internationale: WO9427572

(56) Documents cités:
- EP-A- 0 532 370

## Description

L'invention concerne des compositions cosmétiques contenant au moins un tensio-actif anionique du type alkylgalactoside uronate et au moins un tensio-actif non-ionique du type alkylpolyglycoside et/ou polyglycérolé.

Les compositions de lavage des cheveux ou de la peau sont généralement formulées à partir d'agents tensio-actifs anioniques ou non-ioniques ou leurs mélanges en présence éventuellement d'agents tensio-actifs amphotères.

Les cheveux agressés par les agents atmosphériques tels que la lumière ou les traitements chimiques et lavés avec les compositions de lavage classiques sont difficilement démêlables et cet inconvénient se trouve encore accentué dans le cas de cheveux fins.

Les tensio-actifs anioniques du type alkylgalactoside uronate ont déjà été préconisés dans des compositions lavantes pour les cheveux. Ils ont été décrits dans la demande de brevet EP 0 532 370.

Les agents tensio-actifs non-ioniques de la famille des alkylpolyglycosides ou des polyglycérolés ont également été utilisés dans des compositions lavantes cosmétiques. Ce sont des détergents doux, bien tolérés et biodégradables.

Les compositions de lavage des cheveux utilisant ces tensio-actifs anioniques ou non-ioniques seuls ne conduisent pas à de bonnes propriétés cosmétiques en particulier le démêlage des cheveux mouillés est difficile et les qualités des mousses sont insuffisantes.

La demanderesse vient de découvrir de manière surprenante, que l'association, dans des compositions lavantes et/ou traitantes pour matières kératiniques, d'un tensio-actif anionique du type alkylgalactoside uronate et d'un tensio-actif non-ionique du type alkylpolyglycoside et/ou polyglycérolé confèrait à ces compositions des propriétés de démêlage améliorées.

Par ailleurs, l'association conforme à la présente invention permet d'obtenir une mousse abondante, compacte et très douce.

En outre, la demanderesse a constaté que les compositions cosmétiques contenant une telle association conféraient aux matières kératiniques de bonnes propriétés cosmétiques telles que la douceur, un toucher agréable.

La présente invention a donc pour objet des compositions cosmétiques contenant au moins un agent tensio-actif anionique du type alkylgalactoside uronate et au moins un agent tensio-actif non-ionique du type alkylpolyglycoside et/ou polyglycérolé.

Un autre objet de l'invention est constitué par l'utilisation de ces compositions pour le traitement et/ou le lavage des matières kératiniques telles que les cheveux ou la peau.

Un autre objet concerne un procédé de traitement cosmétique des cheveux ou de la peau au moyen des compositions de l'invention; les procédés de lavage et de traitement des cheveux étant préférés.

Les compositions cosmétiques selon l'invention contiennent dans un milieu aqueux cosmétiquement acceptable :
(**A**) au moins un agent tensio-actif anionique du type alkylgalactoside uronate de formule : 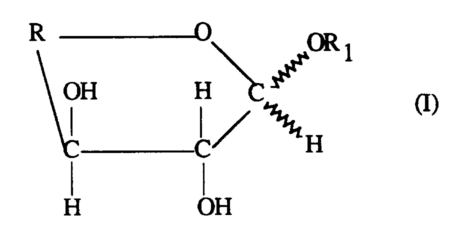 dans laquelle :
   R₁ désigne un radical alkyle linéaire ou ramifié de 8 à 22 atomes de carbone.
   R désigne un groupe
      (i) 〉CH-CH(OH)-CO₂R₂ ou
      (ii) 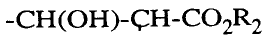 dont le carbone portant le groupe hydroxyle est relié à l'atome d'oxygène endocyclique; R₂ étant un hydrogène, un métal alcalin, un métal alcalino-terreux ou un groupe ammonium quaternaire non substitué ou substitué par des radicaux alkyle, hydroxyalkyle ou dérivé d'amino acides.
(**B**) et au moins un agent tensio-actif non-ionique du type alkylpolyglycoside et/ou polyglycérolé.

Les tensio-actifs anioniques du type alkylgalactoside uronate de formule (I) sont connus et peuvent être préparés selon les procédés décrits dans la demande de brevet EP-A-0 532 370.

Le métal alcalin est notamment le sodium, le potassium et le métal alcalino-terreux est de préférence le magnésium. Comme sels d' ammonium quaternaires, on peut citer les sels d'ammoniaque, de triéthanolamine, de monoéthanolamine, de 2-amino 2-méthyl 1,3-propanediol, de 2-méthyl 2-amino 1-propanol; l'amino acide est notamment l'histidine, l'arginine ou la lysine.

On utilise de préférence les composés de formule (I) pour lesquels le radical R₁ désigne un alkyle en C₈-C₁₄ et plus particulièrement ceux ayant un radical décyle.

On utilise notamment les composés suivants :
Décyl α -D-galactopyranoside uronate de sodium : 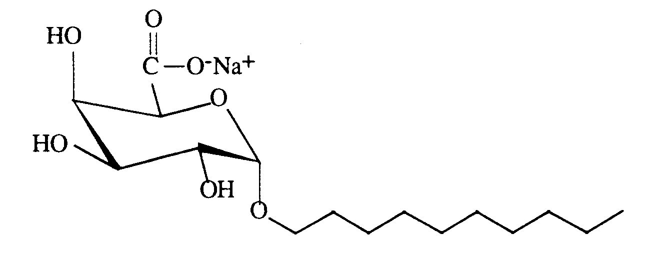
Décyl β -D-galactopyranoside uronate de sodium : 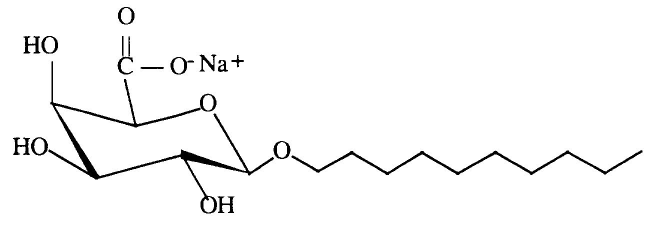
Décyl α-D-galactofuranoside uronate de sodium : 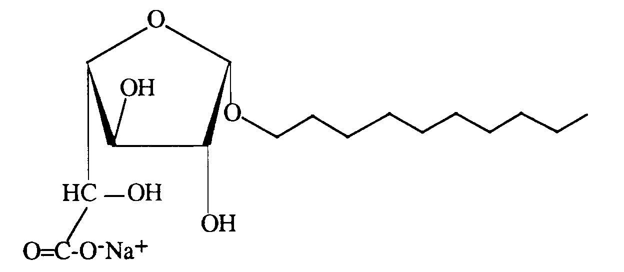
Décylβ -D-galactofuranoside uronate de sodium :

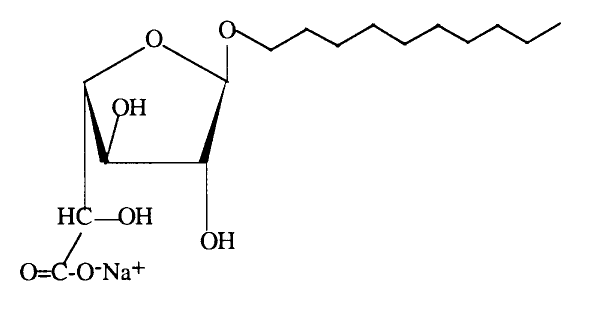

Les alkylpolyglycosides utilisables conformément à l'invention répondent en particulier à la formule (II) suivante

R₁O-(R₂O)ₜ(C₆H₁₀O₅)ₓ―H (II)

correspondant à la stucture développée (III): 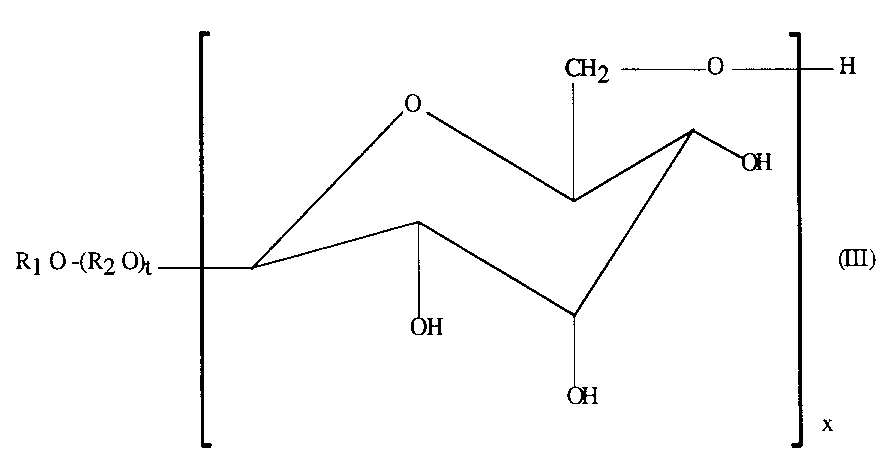 dans laquelle,
R₁ désigne un radical ou un mélange de radicaux alkyles ou alcényles à chaîne droite ou ramifiée en C₈-C₂₄;
R₂ est un radical alkylène en C₂-C₄
t est compris entre 0 et 10 et de préférence entre 0 et 4.
x est un nombre compris entre 1 et 15.

Les composés préférés sont ceux dans lesquels t est égal à 0.

Les composés alkylpolyglycosides de formule développée (III) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; ou sous la dénomination PLANTAREN, tels que les produits PLANTAREN 300, PLANTAREN 600; PLANTAREN 1200 CS/UL ou PLANTAREN 2000 CS/UL les produits vendus par la Société SEPPIC sous les dénominations TRITON CG 110 (ou ORAMIX CG 110) et TRITON CG 312 (ou ORAMIX NS 10); ceux vendus par la Société BASF sous la dénomination LUTENSOL GD 70.

Les agents tensio-actifs non-ioniques du type polyglycérolé utilisés conformément à la présente invention, sont choisis de préférence parmi les composés polyhydroxypropyléthers suivants :
(**A**) les composés répondant à la formule (IV) : 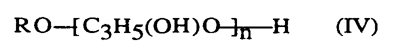 dans laquelle le groupement [C₃H₅ (OH)O] représente les structures suivantes; prises ensemble ou séparément

   ―[CH₂CH OH - CH₂O⁆ (IVa)

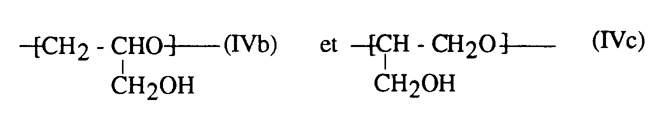

   et R et n ont une des significations ci-après :
   **a**) R représente un radical ou un mélange de radicaux alkyle en C₁₀-C₁₄ et n est un nombre entier ou décimal de 2 à 10 de préférence 3 à 6;
   **b**) R représente un reste :

      R₂CONH CH₂-CH₂OCH₂-CH₂- (V)

      où R₂ désigne un radical ou un mélange de radicaux alkyle et/ou alcényle en C₁₁-C₁₇ et n désigne un nombre entier ou décimal de 1 à 5 et de préférence de 1,5 à 4;
   **c**) R représente un reste :

      R₃ - CHOH - CH₂- (VI)

      où R₃ désigne un radical aliphatique, cycloaliphatique, arylaliphatique en C₇-C₂₁ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.
      Ces tensioactifs de formule (IV) peuvent être préparés selon les procédés décrits dans les brevets FR 1 477 048, 2 328 763 et 2 091 516.
(**B**) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol. Le procédé de préparation de ces composés est décrit dans le brevet FR-A-2 169 787.
(**C**) Les composés polyhydroxypropyléthers, préparés par polyaddition de monochlorhydrine du glycérol sur un composé organique polyhydroxylé en présence d'une base forte, avec élimination au fur et à mesure de l'eau par distillation. Ces composés sont décrits dans le brevet français FR-A-2 574 786.

Parmi les tensio-actifs non ioniques de la famille des polyhydroxypropyléthers décrits dans les paragraphes (**A**), (**B**) et (**C**) ci-dessus, les composés préférés sont représentés par les formules :
(α) 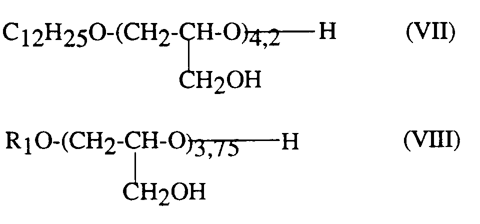 où R₁ désigne un mélange de radicaux alkyles en C₁₀H₂₁ et C₁₂ H₂₅;
(β) les composés préparés par condensation en catalyse alcaline, de 3,5 moles de glycidol sur un alphadiol ayant 12 atomes de carbone, selon le procédé décrit dans le brevet FR-A-2 091 516;
(γ) les composés répondant à la formule :

   R₂-CONH-CH₂CH₂-O-CH₂CH₂-O-(CH₂-CHOH-CH₂-O)_{3,5}H (IX)

   où R₂ désigne un mélange de radicaux comprenant les radicaux alkyle et alcényle suivants :
   C₁₁H₂₃, C₁₃H₂₇, les radicaux dérivés des acides gras du coprah et le radical dérivé de l'acide oléique;
(δ) les composés préparés par condensation de 3,5 moles de glycidol sur un mélange d'alphadiols en C₁₁-C₁₄, décrits dans le brevet FR-A-2 091 516;
(ε) les composés préparés par condensation de 2,5 moles de monochlorhydrine du glycérol sur le dodécanediol -1,2 en présence de soude.

Les alkylgalactoside uronates de formule (I) sont utilisés dans les compositions conformes à l'invention, dans des proportions comprises de préférence entre 0,5 et 30% en poids par rapport au poids total de la composition.

Les tensio-actifs non-ioniques alkylpolyglycosides et/ou polyglycérolés sont utilisés dans les compositions conformes a l'invention dans des proportions comprises de préférence entre 0,5 et 30% en poids par rapport au poids total de la composition.

Si les compositions selon l'invention ne sont pas utilisées pour le lavage des matières kératiniques, la concentration totale en tensio-actifs anioniques de formule (I) et en tensio-actifs non-ioniques alkylpolyglycosides et/ou polyglycérolés est comprise entre 1 et 10% et plus particulièrement entre 1 et 5% en poids par rapport au poids total de la composition. Ces compositions sont utilisées notamment comme compositions à rincer, appliquées avant ou après un shampooing, une coloration, une décoloration, une permanente, un défrisage, dans une composition de coloration, de décoloration, de permanente ou de défrisage.

Lorsque les compositions selon l'invention sont des compositions lavantes, elles contiennent les agents tensio-actifs de formule (I) et les agents tensio-actifs non-ioniques alkylpolyglycosides et/ou polyglycérolés dans une concentration totale comprise entre 5 et 60% en poids et de préférence entre 8 et 40% en poids par rapport au poids total de la composition.

Les compositions peuvent contenir en plus des agents tensio-actifs supplémentaires de nature anionique, non-ionique, amphotère, zwitterionique ou cationique.

Parmi les agents tensio-actifs anioniques, on peut citer les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkylsulfonates, les alkyléthersulfonates, les alkylamides sulfonates, les alkylarylsulfonates, les oléfines sulfonates, les paraffines sulfonates; les alkylsulfosuccinates, les alkyléthersulfosuccinates, les alkylamides sulfosuccinates; les alkylsulfosuccinamates, les alkylsulfoacétates, les alkylétherphosphates; les acylsarcosinates, les acylglutamates, les N-acyltaurates; les iséthionates.

Le radical alkyle ou acyle de ces différents composés est généralement constitué par une chaîne carbonée comportant de 10 à 20 atomes de carbone.

On peut également utiliser des agents tensio-actifs faiblement anioniques, tels que les acides alkylamide ou alkyléthers carboxyliques polyoxyalkylénés, tels que ceux comportant 2 à 50 groupements oxyde d'éthylène.

Les agents tensio-actifs non-ioniques sont plus particulièrement choisis parmi les alcools, les α-diols, les alkylphénols et les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amines grasses polyéthoxylées ayant de préférence 2 à 30 moles d'oxyde d'éthylène; les esters d'acides gras du sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols; les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

On peut également citer les alkylpeptides, les alkylimidazolium bétaïnes.

Parmi les dérivés d'amines, on peut citer les produits commercialisés sous la dénomination "MIRANOL", tels que ceux décrits dans les brevets US-A-2.528.378 et 2.781.354 ou classés dans le dictionnaire CTFA, 3ème édition, 1982, sous les dénominations d'Amphocarboxyglycinates ou d'Amphocarboxypropionates.

Les agents tensio-actifs cationiques sont choisis parmi les sels d'ammonium quaternaires tels que les halogénures d'alkyl(C₈-C₂₂)triméthyl ammonium, les halogénures de dialkyl (C₈-C₂₂) diméthyl ammonium, les halogénures d'alkyl(C₈-C₂₂) diméthyl hydroxyéthyl ammonium.

Les co-tensio-actifs additionnels, peuvent représenter jusqu'à 50 % du poids total des agents tensio-actifs présents dans la composition.

Le pH des compositions conformes à l'invention est généralement compris entre 2 et 10,5, plus particulièrement entre 3 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C₁-C₄ comme l'éthanol, l'isopropanol, le n-butanol; les alkylèneglycols comme le propylèneglycol; les éthers de glycols.

Les compositions selon l'invention peuvent se présenter sous forme de liquide plus ou moins épaissi, de gel, d'émulsion (lait ou crème), de lotion hydroalcoolique, de dispersion, de pain solide ou de mousse aérosol.

Les compositions sont par exemple des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, des vernis à ongles, des fards et fonds de teint pour le visage, des shampooings, des produits pour le bain ou la douche, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions conformes à l'invention peuvent également contenir en plus divers additifs tels des agents épaississants tels que des acides polyacryliques, des dérivés de cellulose, des esters d'acides gras et de polyéthylèneglycol; des séquestrants; des renforçateurs de mousse; des conservateurs; des parfums; des électrolytes; des corps gras tels que des alcools gras, des céramides, des huiles ou cires minérales, végétales, animales ou synthétiques; des filtres UV; des agents anti-radicaux libres; des agents nacrants; des biocides; des antibactériens; des agents anti-pelliculaires; des agents anti-séborthéïques; des anti-parasitaires; des repellents; des colorants; des pigments; des oxydants; des réducteurs; des hydratants; des polymères anioniques, cationiques, non ioniques ou amphotères; des vitamines; des α-hydroxyacides.

Le traitement des matières kératiniques se fait par application sur ces matières d'une quantité cosmétiquement acceptable d'une composition telle que définie ci-dessus.

Le procédé de lavage et/ou de conditionnement des matières kératiniques et en particulier des cheveux ou de la peau conforme à l'invention consiste à appliquer sur ces matières au moins une composition telle que définie ci-dessus, cette application étant suivie éventuellement d'une étape de rinçage à l'eau.

Les compositions de lavage peuvent être utilisées comme shampooing mais également comme gel-douche pour le lavage des cheveux et de la peau, auquel cas ils sont appliqués sur la peau et les cheveux humides qui sont rincés après application.

Lorsque les compositions sont utilisées pour le conditionnement des cheveux, elles sont appliquées sur les cheveux humides, après quoi on peut soit les sécher soit après un temps de pose de 1 à 10 minutes, les rincer à l'eau. On constate que les cheveux humides se démêlent bien.

Les exemples qui suivent sont destinés à illustrer l'invention sans pour autant présenter un caractère limitatif.

### EXEMPLE 1

| **APRES SHAMPOOING** | |
|---|---|
| - Décyl - D-galactoside uronate de sodium | 2 g MA |
| - Dodécanediol polyglycérolé à 3,5 moles de glycérol | 1 g MA |
| - Mélange d'alcools cétylstéarylique et cétylstéarylique oxyéthyléné (33 OE) 80/20 vendu sous le nom DEHSCONET 390 par Tensia | 5 g |
| - Chlorure de distéaryl diméthyl ammonium | 3 g |
| Colorants, parfum, conservateur | |
| eau qsp | |
| pH ajusté à 5 par HCl | |

La composition se présente sous la forme d'une crème blanche fluide.

### EXEMPLE 2

| **BAIN MOUSSANT** | |
|---|---|
| - Décyl - D-galactoside uronate de sodium | 25 g MA |
| - Alkyl (C₉/C₁₀/C₁₁ 20/40/40)polyglucoside (1,4) en solution aqueuse à 50 %, vendu sous le nom d'APG 300 par la Société HENKEL | 5 g MA |
| - Ethers d'hexadécanediol (3 moles) et de polyéthylène glycol 60 OE | 5 g |
| Colorants, parfum, conservateur | |
| eau qsp | 100 g |
| pH ajusté à 6 par HCl. | |

La composition présente un aspect limpide et visqueux.

### EXEMPLE 23

| **GEL DOUCHE** | |
|---|---|
| - Décyl -D-galactoside uronate de sodium | 15 g MA |
| - Dodécanediol polyglycérolé à 3,5 moles de glycérol | 10 g MA |
| - Glycérine pure | 2 g |
| - Suif oxyéthyléné (60 OE) Ether de myristil glycol vendu sous le nom d'Elfacos GT 282 S par la Société AKZO | 3 g |
| Colorants, parfum, conservateur | |
| eau qsp | |
| pH ajusté à 7,5 par NaOH. | |

La composition présente un aspect limpide et visqueux.

### EXEMPLE 4

| **SHAMPOOING** | |
|---|---|
| - Décyl -D-galactoside uronate de sodium | 7,5 g MA |
| - Alkyl (C₉/C₁₀/C₁₁ 20/40/40)polyglucoside 1,4) en solution aqueuse à 50 %, vendu sous le nom d'APG 300 par la Société HENKEL | 7,5 g MA |
| - Diuréthane d'alcools (C16/C18)oxyéthyléné et oxypropyléné vendu sous le nom de Dapral T 212 par la société AKZO | 3 g |
| Colorants, parfum, conservateur | |
| eau qsp | 100 g |
| pH ajusté à 6,4 par HCl. | |

La composition présente un aspect limpide et visqueux.

## Revendications

1. Composition cosmétique, caractérisé par le fait qu'elle contient dans un milieu aqueux cosmétiquement acceptable :
(**A**) au moins un agent tensio-actif anionique du type alkylgalactoside uronate de formule : 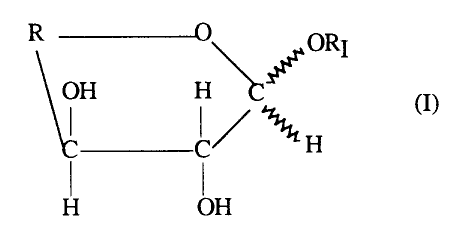
R₁ désigne un radical alkyle linéaire ou ramifié de 8 à 22 atomes de carbone.
R désigne un groupe
(i) 〉CH-CH(OH)-CO₂R₂ ou
(ii) 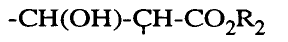 dont le carbone portant le groupe hydroxyle est relié à l'atome d'oxygène endocyclique; R₂ étant un hydrogène, un métal alcalin, un métal alcalin-terreux ou un groupe ammonium quaternaire non substitué ou substitué par des radicaux alkyle, hydroxyalkyle ou dérivé d'amino acide.
(**B**) au moins un agent tensio-actif non-ionique du type alkylpolyglycoside et/ou polyglycérolé.

2. Composition selon la revendication 1, caractérisée par le fait que dans la formule (I), le radical R₂ désigne le sodium ou le potassium; le magnésium; le groupe ammonium quaternaire dérivé d'ammoniaque, de triéthanolamine, de monoéthanolamine, de 2-amino 2-méthyl 1,3-propanediol, de 2-méthyl 2-amino 1-propanol, d'histidine, d'arginine ou de lysine.

3. Composition selon la revendication 1 ou 2, caractérisée par le fait que le composé de formule (I) est choisi parmi ceux pour lesquels R₁ désigne un alkyle en C₈-C₁₄.

4. Composition selon l'une quelconque des revendications 1 à 3, caractérisée par le fait que les composés de formule (I) sont choisis parmi ceux pour lesquels R₁ désigne un radical décyle.

5. Composition selon la revendication 4, caractérisée par le fait que le composé de formule (I) est choisi parmi :
le décyl α-D-galactopyranoside uronate de sodium
le décyl β-D-galactopyranoside uronate de sodium
le décyl α-D-galactofuranoside uronate de sodium
le décyl β-D-galactofuranoside uronate de sodium.

6. Composition selon l'une quelconque des revendications 1 à 4, caractérisée en ce que les agents tensio-actifs non-ioniques de la famille des alkylglycosides répondent à la formule suivante :
R₁O-(R₂O)ₜ(C₆H₁₀―O₅)ₓ―H (II)
correspondant à la stucture développée suivante : 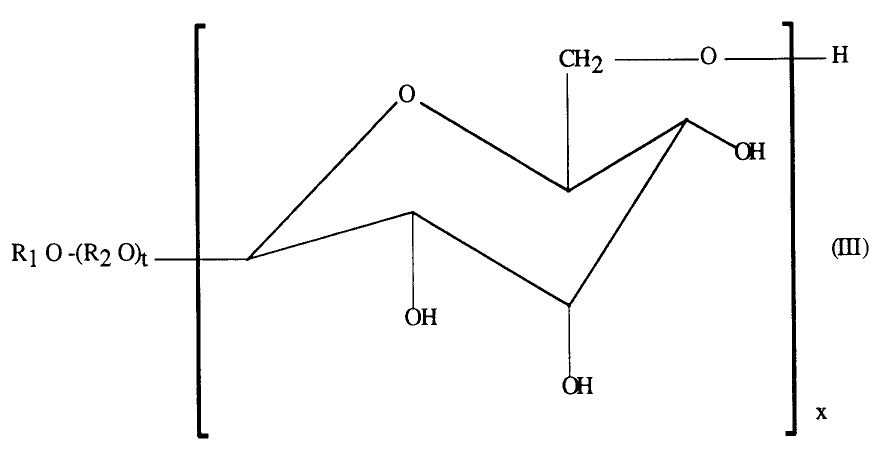 dans laquelle,
R₁ désigne un radical ou un mélange de radicaux alkyles ou alcényles à chaîne droite ou ramifiée en C₈-C₂₄;
R₂ désigne un radical alkylène en C₂-C₄
x est un nombre compris entre 1 et 15
t est un nombre compris entre 0 et 10 et de préférence entre 0 et 4.

7. Composition selon l'une des revendications 1 à 6, caractérisée en ce que les agents tensio-actis non-ioniques polyglycérolés sont choisis parmi les polyhydroxypropyléthers suivants :
(**A**) les composés répondant à la formule (IV) : 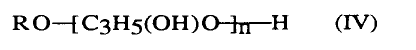 dans laquelle le groupement [C₃H₅ (OH)O] représente les structures suivantes, prises ensemble ou séparément :
―[CH₂CH OH - CH₂O⁆ (IVa)
et 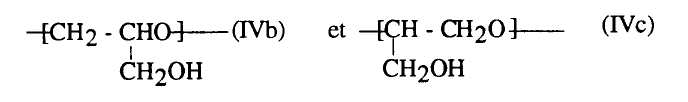 et R et n ont une des significations ci-après :
**a**) R représente un radical ou un mélange de radicaux alkyle en C₁₀-C₁₄ et n est un nombre entier ou décimal de 2 à 10 de préférence 3 à 6;
**b**) R représente un reste :
R₂CONH CH₂-CH₂OCH₂-CH₂- (V)
où R₂ désigne un radical ou un mélange de radicaux alkyle et/ou alcényle en C₁₁-C₁₇ et n désigne un nombre entier ou décimal de 1 à 5 et de préférence de 1,5 à 4;
**c**) R représente un reste :
R₃ - CHOH - CH₂- (VI)
où R₃ désigne un radical aliphatique, cycloaliphatique, arylaliphatique en C₇-C₂₁ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10;
(**B**) les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol;
(**C**) les composés polyhydroxypropyléthers, préparés par polyaddition de monochlorhydrine du glycérol sur un composé organique polyhydroxylé en présence d'une base forte, avec élimination au fur et à mesure de l'eau par distillation.

8. Composition selon la revendication 7, caractérisée en ce que les agents tensio-actifs non-ioniques polyglycérolés sont choisis parmi les polyhydroxypropyléthers suivants :
(α) 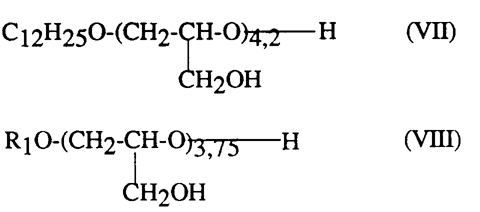 où R₁ désigne un mélange de radicaux alkyles en C₁₀H₂₁ et C₁₂ H₂₅;
(β) les composés préparés par condensation en catalyse alcaline, de 3,5 moles de glycidol sur un alphadiol ayant 12 atomes de carbone;
(γ) les composés répondant à la formule :
R₂-CONH-CH₂CH₂-O-CH₂CH₂-O-(CH₂-CHOH-CH₂-O)_{3,5} H (IX)
où R₂ désigne un mélange de radicaux comprenant les radicaux alkyle et alcényle suivants :
C₁₁H₂₃, C₁₃H₂₇, les radicaux dérivés des acides gras du coprah et le radical dérivé de l'acide oléique;
(δ) les composés préparés par condensation de 3,5 moles de glycidol sur un mélange d'alphadiols en C₁₁-C₁₄.
(ε) les composés préparés par condensation de 2,5 moles de monochlorhydrine du glycérol sur le dodécanediol-1,2.

9. Composition selon l'une quelconque des revendications 1 à 8, caractérisée par le fait que les agents tensio-actifs anioniques de formule (I) sont présents dans des proportions comprises entre 0,5 et 30% en poids et les agents tensio-actifs non-ioniques alkyl polyglycosides et/ou polyglycérolés sont présents dans des proportions comprises entre 0,5 et 30% en poids; les pourcentages en poids étant exprimés par rapport au poids total de la composition.

10. Composition de conditionnement des matières kératiniques selon l'une quelconque des revendications 1 à 9, caractérisé par le fait que la concentration totale en agents tensio-actifs anioniques de formule (I) et en agents tensio-actifs non-ioniques alkyl polyglycosides et/ou polyglycérolés est comprise entre 1 et 10% en poids par rapport au poids total de la composition.

11. Composition de lavage des matières kératiniques selon l'une quelconque des revendications 1 à 9, caractérisée par le fait que la concentration totale en tensio-actifs anioniques de formule (I) et en tensio-actifs non-ioniques alkylpolyglycosides et/ou polyglycérolés est comprise entre 5 et 60% en poids par rapport au poids total de la composition.

12. Composition selon l'une quelconque des revendications 1 à 11, caractérisée par le fait qu'elle contient en plus un co-tensio-actif additionnel du type anionique, non-ionique, amphotère ou cationique dans une proportion allant jusqu'à 50% du poids total en agents tensio-actifs.

13. Composition selon la revendication 12, caractérisée par le fait que le co-tensio-actif anionique additionnel est choisi parmi les sels alcalins, les sels d'ammonium, les sels d'amminoalcools, les sels de magnésium des composés suivants : acides gras, alkylsulfates, alkyléthersulfates, alkylamidoéthersulfates, alkylarylpolyéthersulfates, monoglycérides sulfates; les alkylsulfonates, alkyléthersulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates, les acylglutamates, les N-acyltaurates; les iséthionates; le radical alkyle ou acyle étant constitué d'une chaîne carbonée de 10 et 20 atomes de carbone ou bien des acides alkylamides ou alkyléthers carboxyliques polyoxyalkylénés.

14. Composition selon la revendication 12, caractérisée par le fait que le co-tensio-actif non-ionique additionnel est choisi parmi les alcools, les α-diols, les alkylphénols et les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30, les copolymères d'oxydes d'éthylène et de propylène; les condensats d'oxydes d'éthylène et de propylène sur des alcools gras, les amides gras polyéthoxylés; les amines grasses polyéthoxylées; les esters d'acides gras du sorbitan oxyéthylénés, les esters d'acides gras de sucre; les esters d'acides gras de polyéthylèneglycols; les esters gras de glycols; les oxydes d'amines.

15. Composition selon la revendicaton 12, caractérisée par le fait que le co-tensio-actif amphotère additionnel est choisi parmi les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes; les alkylpeptides; les alkylimidazolium bétaïnes.

16. Composition selon la revendication 13, caractérisée par le fait que le co-tensio-actif cationique est choisi parmi les sels d'ammonium quaternaire.

17. Composition selon l'une quelconque des revendications 1 à 16, caractérisée par le fait que le milieu cosmétiquement acceptable est constitué par de l'eau ou un mélange d'eau et d'un solvant cosmétiquement acceptable.

18. Composition selon l'une quelconque des revendications 1 à 17, caractérisée par le fait qu'elle se présente sous forme de liquide plus ou moins épaissi, de gel, d'émulsion, de lotion hydroalcoolique, de dispersion, de pain solide ou de mousse aérosol.

19. Composition selon l'une quelconque des revendications 1 à 18, caractérisée par le fait qu'elle contient en plus des additifs choisis parmi les renforçateurs de mousse, les épaississants, les séquestrants, les électrolytes, les parfums, les conservateurs, les alcools gras, les huiles ou cires minérales, végétales, animales ou synthétiques, les céramides, les filtres UV, les agents anti-radicaux libres, les agents nacrants, les biocides, les antibactériens, les agents anti-pelliculaires, les agents anti-séborrhéïques, les anti-parasitaires, les repellents, les colorants, les pigments, les oxydants, des réducteurs, les hydratants, les polymères anioniques, cationiques ou non-ioniques amphotères, les vitamines, les α-hydroxyacides.

20. Utilisation de la composition telle que définie dans l'une quelconque des revendications 1 à 19 pour le traitement et/ou le lavage des matières kératiniques, particulièrement des cheveux et/ou de la peau.

21. Procédé de lavage et/ou de conditionnement cosmétique des cheveux ou de la peau, caractérisé par le fait qu'il consiste à appliquer sur la peau ou les cheveux une quantité efficace de composition selon l'une quelconque des revendications 1 à 19, cette application étant éventuellement suivie d'un rinçage à l'eau.

## Claims

1. Cosmetic composition, characterized in that it contains, in a cosmetically acceptable agueous medium:
**(A)** at least one anionic surfactant of alkylgalactoside uronate type of formula: 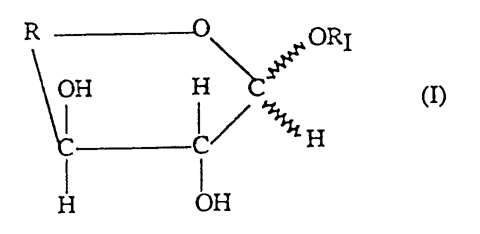 [lacuna]
R₁ denotes a linear or branched alkyl radical containing 8 to 22 carbon atoms,
R denotes a group
(i) 〉CH-CH(OH)-CO₂R₂ or
(ii) 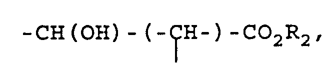 in which the carbon carrying the hydroxyl group is connected to the endocyclic oxygen atom; R₂ being a hydrogen, an alkali metal, an alkaline-earth metal or a quaternary ammonium group which is unsubstituted or substituted by alkyl or hydroxyalkyl radicals or derived from amino acid,
**(B)** at least one nonionic surfactant of alkylpolyglycoside and/or polyglycerolated type.

2. Composition according to Claim 1, characterized in that, in the formula (I), the radical R₂ denotes sodium or potassium; magnesium; or the quaternary ammonium group derived from ammonia, triethanolamine, monoethanolamine, 2-amino-2-methyl-1,3-propanediol, 2-methyl-2-amino-1-propanol, histidine, arginine or lysine.

3. Composition according to Claim 1 or 2, characterized in that the compound of formula (I) is chosen from those in which R₁ denotes a C₈-C₁₄ alkyl.

4. Composition according to any one of Claims 1 to 3, characterized in that the compounds of formula (I) are chosen from those in which R₁ denotes a decyl radical.

5. Composition according to Claim 4, characterized in that the compound of formula (I) is chosen from:
sodium decyl α-D-galactopyranoside uronate
sodium decyl β-D-galactopyranoside uronate
sodium decyl α-D-galactofuranoside uronate
sodium decyl β-D-galactofuranoside uronate.

6. Composition according to any one of Claims 1 to 4, characterized in that the nonionic surfactants of the alkylglycoside family correspond to the following formula:
R₁O-(R₂O)ₜ(C₆H₁₀―O₅)ₓ―H (II)
corresponding to the following expanded structure: 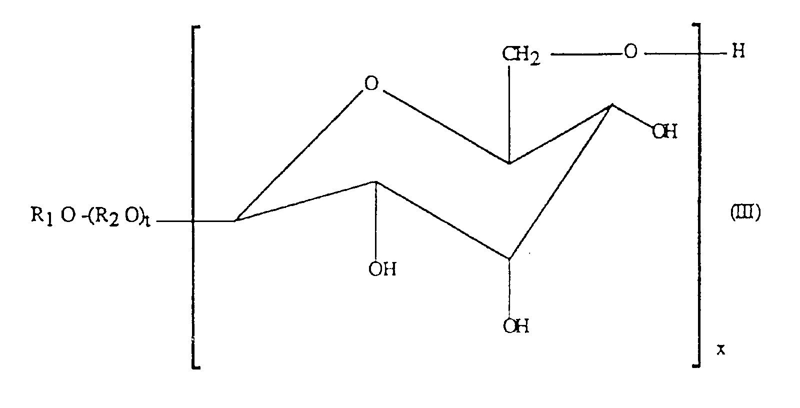 in which,
R₁ denotes a C₈-C₂₄, straight- or branched-chain alkyl or alkenyl radical or a mixture of C₈-C₂₄, straight- or branched-chain alkyl or alkenyl radicals,
R₂ denotes a C₂-C₄ alkylene radical,
x is a number between 1 and 15,
t is a number between 0 and 10 and preferably between 0 and 4.

7. Composition according to one of Claims 1 to 6, characterized in that the nonionic polyglycerolated surfactants are chosen from the following polyhydroxypropyl ethers:
(**A**) the compounds corresponding to the formula (IV): 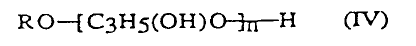 in which the [C₃H₅(OH)O] group represents the following structures; taken together or separately:
―[CH₂CH OH - CH₂O⁆ (IVa)
and 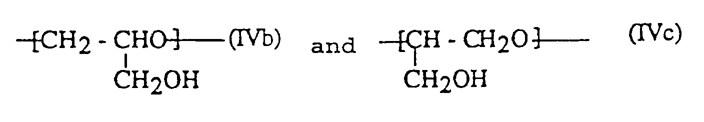 and R and n have one of the meanings hereinbelow:
**a**) R represents a C₁₀-C₁₄ alkyl radical or a mixture of C₁₀-C₁₄ alkyl radicals and n is a whole or decimal number from 2 to 10, preferably 3 to 6;
**b**) R represents a residue:
R₂CONHCH₂-CH₂OCH₂-CH₂- (V)
where R₂ denotes a C₁₁-C₁₇ alkyl and/or alkenyl radical or a mixture of C₁₁-C₁₇ alkyl and/or alkenyl radicals and n denotes a whole or decimal number from 1 to 5 and preferably from 1.5 to 4;
**c**) R represents a residue:
R₃-CHOH-CH₂- (VI)
where R₃ denotes a C₇-C₂₁ aliphatic, cycloaliphatic or arylaliphatic radical and their mixtures, the aliphatic chains denoting in particular alkyl chains which can contain from 1 to 6 ether, thioether and/or hydroxymethylene groups, and n denotes a whole or decimal number from 1 to 10;
(**B**) the compounds prepared by condensation, using acid catalysis, of 2 to 10, and preferably of 2.5 to 6, mol of glycidol per mole of alcohol or of alpha-diol containing 10 to 14 carbon atoms, at a temperature of 50 to 120°C, the glycidol being slowly added to the alcohol or to the alpha-diol;
(**C**) the polyhydroxypropyl ether compounds prepared by polyaddition of glycerol monochlorohydrin to a polyhydroxylated organic compound in the presence of a strong base with removal, by distillation, of the water as it is formed.

8. Composition according to Claim 7, characterized in that the nonionic polyglycerolated surfactants are chosen from the following polyhydroxypropyl ethers:
(α) 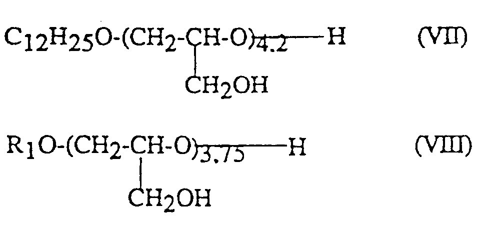 where R₁ denotes a mixture of C₁₀H₂₁ and C₁₂H₂₅ alkyl radicals;
(β) the compounds prepared by condensation, using alkaline catalysis, of 3.5 mol of glycidol with an alpha-diol having 12 carbon atoms;
(γ) the compounds corresponding to the formula:
R₂-CONH-CH₂CH₂-O-CH₂CH₂-O-(CH₂-CHOH-CH₂-O)_{3.5}H (IX)
where R₂ denotes a mixture of radicals comprising the following alkyl and alkenyl radicals:
C₁₁H₂₃, C₁₃H₂₇, the radicals derived from coconut fatty acids and the radical derived from oleic acid;
(δ) the compounds prepared by condensation of 3.5 mol of glycidol with a mixture of C₁₁-C₁₄ alpha-diols;
(ε) the compounds prepared by condensation of 2.5 mol of glycerol monochlorohydrin with 1,2-dodecanediol.

9. Composition according to any one of Claims 1 to 8, characterized in that the anionic surfactants of formula (I) are present in proportions of between 0.5 and 30% by weight and the nonionic alkylpolyglycoside and/or polyglycerolated surfactants are present in proportions of between 0.5 and 30% by weight; the percentages by weight being expressed with respect to the total weight of the composition.

10. Composition for conditioning keratinous substances according to any one of Claims 1 to 9, characterized in that the total concentration of anionic surfactants of formula (I) and of nonionic alkylpolyglycoside and/or polyglycerolated surfactants is between 1 and 10% by weight with respect to the total weight of the composition.

11. Composition for washing keratinous substances according to any one of Claims 1 to 9, characterized in that the total concentration of anionic surfactants of formula (I) and of nonionic alkylpolyglycoside and/or polyglycerolated surfactants is between 5 and 60% by weight with respect to the total weight of the composition.

12. Composition according to any one of Claims 1 to 11, characterized in that it furthermore contains an additional cosurfactant of anionic, nonionic, amphoteric or cationic type in a proportion ranging up to 50% of the total weight of surfactants.

13. Composition according to Claim 12, characterized in that the additional anionic cosurfactant is chosen from the alkali metal salts, the ammonium salts, [lacuna] the aminoalcohol salts or the magnesium salts of the following compounds: fatty acids, alkyl sulfates, alkyl ether sulfates, alkylamidoether sulfates, alkylarylpolyether sulfates or monoglyceride sulfates; the alkylsulfonates, alkylethersulfonates, alkylamidesulfonates, alkylarylsulfonates, olefinsulfonates or paraffinsulfonates; the alkylsulfosuccinates, alkylethersulfosuccinates or alkylamidesulfosuccinates; the alkylsulfosuccinamates; the alkylsulfoacetates; the alkyl ether phosphates; the acylsarcosinates, acylglutamates or N-acyltaurates; or the isethionates; the alkyl or acyl radical consisting of a carbon chain containing 10 and [sic] 20 carbon atoms or else polyoxyalkylenated alkyl amide or alkyl ether carboxylic acids.

14. Composition according to Claim 12, characterized in that the additional nonionic cosurfactant is chosen from the polyethoxylated or polypropoxylated alcohols, α-diols, alkylphenols and fatty acids, with a fatty chain containing 8 to 18 carbon atoms, the number of ethylene oxide or propylene oxide groups being between 2 and 50 and the number of glycerol groups being between 2 and 30 [sic], the copolymers of ethylene oxide and of propylene oxide; the condensates of ethylene oxide and of propylene oxide with fatty alcohols, the polyethoxylated fatty amides; the polyethoxylated fatty amines; the oxyethylenated fatty acid eaters of sorbitan, the fatty acid esters of sugar [sic]; the fatty acid eaters of polyethylene glycols; the fatty acid eaters of glycols; or the amine oxides.

15. Composition according to Claim 12, characterized in that the additional amphoteric cosurfactant is chosen from the derivatives of secondary or tertiary aliphatic amines, in which the aliphatic radical is a linear or branched chain containing 8 to 18 carbon atoms and which contains at least one water-solubilizing carboxylate, sulfonate, sulfate, phosphate or phosphonate anionic group; the (C₈-C₂₀)alkylbetaines, the sulfobetaines, the (C₈-C₂₀)alkylamido(C₁-C₆)alkylbetaines or the (C₈-C₂₀)-alkylamido(C₁-C₆)alkylsulfobetaines; the alkylpeptides; or the alkylimidazolium betaines.

16. Composition according to Claim 13, characterized in that the cationic cosurfactant is chosen from the quaternary ammonium salts.

17. Composition according to any one of Claims 1 to 16, characterized in that the cosmetically acceptable medium consists of water or a mixture of water and of a cosmetically acceptable solvent.

18. Composition according to any one of Claims 1 to 17, characterized in that it is provided in the form of a more or less thickened liquid, a gel, an emulsion, an aqueous/alcoholic lotion, a dispersion, a solid bar or an aerosol foam.

19. Composition according to any one of Claims 1 to 18, characterized in that it furthermore contains additives chosen from foam reinforcers, thickeners, sequestering agents, electrolytes, fragrances, preservatives, fatty alcohols, mineral, vegetable, animal or synthetic oils or waxes, ceramides, UV screening agents, agents for combating free radicals, pearlescence agents, biocides, antibacterials, antidandruff agents, anti-seborrheic agents, antiparasitic agents, repellents, dyes, pigments, oxidizing agents, reducing agents, moisturizers, anionic, cationic or nonionic amphoteric [sic] polymers, vitamins or α-hydroxy acids.

20. Use of the composition as defined in any one of Claims 1 to 19 for treating and/or washing keratinous substances, particularly the hair and/or the skin.

21. Process for cosmetic washing and/or conditioning of the hair or of the skin, characterized in that it consists in applying an effective amount of composition according to any one of Claims 1 to 19 to the skin or the hair, this application optionally being followed by a rinsing with water.

## Patentansprüche

1. Kosmetische Zusammensetzung,
dadurch **gekennzeichnet**, daß
sie in einem wässrigen, kosmetisch geeigneten Medium enthält:
(A) mindestens ein anionisches oberflächenaktives Mittel vom Alkylgalactosiduronat-Typ der Formel: 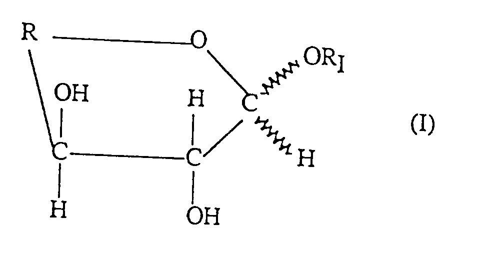 worin gilt:
R₁ bedeutet einen linearen oder verzweigten Alkylrest mit 1 bis 22 Kohlenstoffatomen;
R bedeutet eine Gruppe:
(i) 〉CH-CH(OH)-CO₂R₂ oder
(ii) 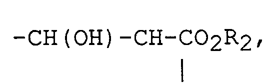 worin der Kohlenstoff mit der Carboxylgruppe an das endozyklische Sauerstoffatom gebunden ist, und worin R₂ ein Wasserstoffatom, ein Alkali- oder Erdalkalimetallatom oder eine gegebenenfalls mit Alkyl- oder Hydroxyalkylresten substituierte quaternäre Ammoniumgruppe oder eine von Aminosäuren abgeleitete Gruppe ist; sowie
(B) mindestens ein nicht-ionisches oberflächenaktives Mittel des Typs von Alkylpolyglycosid- und/oder Polyglycerylverbindungen.

2. Zusammensetzung gemäß Anspruch 1,
dadurch **gekennzeichnet**, daß
in der Formel (I) der Rest R₂ Natrium oder Kalium, Magnesium oder eine quaternäre Ammoniumgruppe bedeutet, die von Ammoniak, Triethanolamin, Monoethanolamin, 2-Amino-2-methylpropan-1,3-diol, 2-Methyl-2-aminopropan-1-ol, Hystidin, Arginin oder Lysin abgeleitet ist.

3. Zusammensetzung gemäß Anspruch 1 oder 2,
dadurch **gekennzeichnet**, daß
die Verbindung der Formel (I) aus denjenigen ausgewählt ist, für welche R₁ einen C₈₋₁₄-Alkylrest bedeutet.

4. Zusammensetzung gemäß einem der Ansprüche 1 bis 3,
dadurch **gekennzeichnet**, daß
die Verbindungen der Formel (I) aus denjenigen ausgewählt ist, für welche R₁ einen Decylrest bedeutet.

5. Zusammensetzung gemäß Anspruch 4,
dadurch **gekennzeichnet**, daß
die Verbindung der Formel (I) ausgewählt ist aus:
Natrium-Decyl-α-D-galactopyranosiduronat,
Natrium-Decyl-β-D-galactopyranosiduronat,
Natrium-Decyl-α-D-galactofuranosiduronat, und aus
Natrium-Decyl-β-D-galactofuranosiduronat.

6. Zusammensetzung gemäß einem der Ansprüche 1 bis 4,
dadurch **gekennzeichnet**, daß
die nicht-ionischen oberflächenaktiven Mittel der Familie der Alkylglycoside die folgende Formel aufweisen:
R₁O-(R₂O)ₜ(C₆H₁₀―O₅)ₓ―H (II)
entsprechend der folgenden Strukturformel: 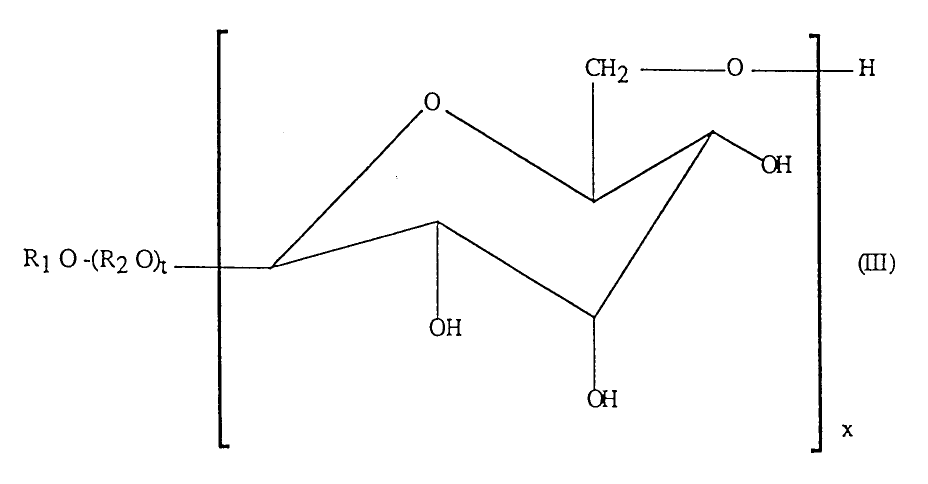 worin gilt:
R₁ bedeutet einen geradkettigen oder verzweigten C₈₋₂₄-Alkyl- oder -Alkenylrest oder eine Mischung dieser Reste;
R₂ bedeutet einen C₂₋₄-Alkylenrest;
x ist eine Zahl von 1 bis 15;
t beträgt 0 bis 10 und vorzugsweise 0 bis 4.

7. Zusammensetzung gemäß einem der Ansprüche 1 bis 6,
dadurch **gekennzeichnet**, daß
die nicht-ionischen Polyglyceryl-Tenside aus den folgenden Polyhydroxypropylethern ausgewählt sind:
(A) aus Verbindungen der Formel (IV): 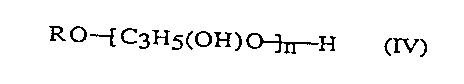 worin die Gruppierung (C₃H₅(OH)O), jeweils zusammen oder einzeln, die folgenden Strukturen darstellt:
―[CH₂CH OH-CH₂O⁆ (IVa)
und 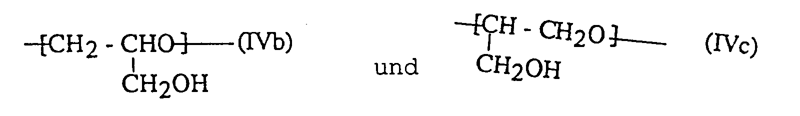 und worin R und n die folgenden Bedeutungen haben:
(a) R bedeutet einen C₁₀₋₁₄-Alkylrest oder eine Mischung dieser Reste, und n ist eine ganze Zahl oder Dezimalzahl von 2 bis 10 und vorzugsweise von 3 bis 6;
b) R stellt einen Rest dar:
R₂CONH CH₂-CH₂OCH₂-CH₂- (V)
worin R₂ einen C₁₁₋₁₇-Alkyl- und/oder -Alkenylrest oder eine Mischung dieser Reste und n eine ganze Zahl oder Dezimalzahl von 1 bis 5 und vorzugsweise von 1,5 bis 4 bedeuten;
c) R stellt einen Rest dar:
R₃-CHOH-CH₂- (VI)
worin R₃ einen aliphatischen, cycloaliphatischen oder arylaliphatischen C₇₋₂₁-Rest oder deren Mischungen bedeutet, wobei die aliphatischen Ketten insbesondere Alkylketten bedeuten, die 1 bis 6 Ether-, Thioether- und/oder Hydroxymethylen-Gruppierungen aufweisen können, und n bedeutet eine ganze Zahl oder Dezimalzahl von 1 bis 10;
(B) aus Verbindungen, die durch Kondensation, unter saurer Katalyse, von 2 bis 10 und vorzugsweise von 2,5 bis 6 Mol Glycidol pro Mol Alkohol oder α-Diol mit 10 bis 14 Kohlenstoffatomen bei einer Temperatur von 50 bis 120°C hergestellt sind, wobei das Glycidol langsam zum Alkohol oder α-Diol gegeben wird;
(C) aus Polyhydroxypropyletherverbindungen, die durch Polyaddition des Monochlorhydrins von Glycerin an eine organische polyhydroxylierte Verbindung in Gegenwart einer starken Base unter destillativer Beseitigung von Wasser hergestellt sind.

8. Zusammensetzung gemäß Anspruch 7,
dadurch **gekennzeichnet**, daß
die nicht-ionischen Polyglyceryl-Tenside aus den folgenden Polyhydroxypropylethern ausgewählt sind:
(α) 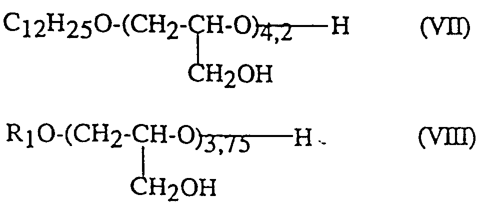 worin R₁ eine Mischung aus C₁₀H₂₁- und C₁₂H₂₅-Alkylresten bedeutet;
(β) aus Verbindungen, die durch unter alkalischer Katalyse durchgeführte Kondensation von 3,5 Mol Glycidol an ein α-Diol mit 12 Kohlenstoffatomen hergestellt sind;
(γ) aus Verbindungen der Formel:
R₂-CONH-CH₂CH₂-O-CH₂CH₂-O-(CH₂-CHOH-CH₂-O)_{3,5} H (IX)
worin R₂ eine Mischung aus Resten bedeutet, die die folgenden Alkyl- und Alkenylreste umfassen:
C₁₁H₂₃, C₁₃H₂₇, von Fettsäuren von Kopra abgeleitete Reste und den von Ölsäure abgeleiteten Rest;
(δ) aus Verbindungen, die durch Kondensation von 3,5 Mol Glycidol an eine Mischung aus C₁₁₋₁₄-α-Diolen hergestellt sind;
(ε) aus Verbindungen, die durch Kondensation von 2,5 Mol Monochlorhydrin von Glycerin an Dodecandiol-1,2 hergestellt sind.

9. Zusammensetzung gemäß einem der Ansprüche 1 bis 8,
dadurch **gekennzeichnet**, daß
die anionischen oberflächenaktiven Mittel der Formel (I) in Mengenanteilen von 0,5 bis 30 Gew.% und die nicht-ionischen Alkylpolyglycosid- und/oder polyglycerierten Tenside in Mengenanteilen von 0,5 bis 30 Gew.% vorhanden sind, wobei die Gew.%-Angaben auf das Gesamtgewicht der Zusammensetzung bezogen sind.

10. Zusammensetzung gemäß jedem der Ansprüche 1 bis 9 zum Konditionieren keratinischer Materien,
dadurch **gekennzeichnet**, daß
die Gesamtkonzentration an anionischen Tensiden der Formel (I) und an nicht-ionischen Alkylpolyglycosid- und/oder polyglycerierten Tensiden 1 bis 10 Gew.% beträgt, bezogen auf das Gesamtgewicht der Zusammensetzung.

11. Zusammensetzung gemäß jedem der Ansprüche 1 bis 9 zum Waschen keratinischer Materien,
dadurch **gekennzeichnet**, daß
die Gesamtkonzentration an anionischen Tensiden der Formel (I) und an nicht-ionischen Alkylpolyglycosid- und/oder polyglycerierten Tensiden 5 bis 60 Gew.% beträgt, bezogen auf das Gesamtgewicht der Zusammensetzung.

12. Zusammensetzung gemäß jedem der Ansprüche 1 bis 11,
dadurch **gekennzeichnet**, daß
sie zusätzlich ein weiteres Co-Tensid vom anionischen, nichtionischen, amphoteren oder kationischen Typ in einem Mengenanteil bis zu 50% des Gesamtgewichts an oberflächenaktiven Mitteln enthält.

13. Zusammensetzuung gemäß Anspruch 12,
dadurch **gekennzeichnet**, daß
das anionische zusätzliche Co-Tensid aus Alkali-, Ammonium-, Aminoalkohol- und Magnesiumsalzen der folgenden Verbindungen ausgewählt ist: von Fettsäuren, Alkylsulfaten, Alkylethersulfaten, Alkylamidoethersulfaten, Alkylarylpolyethersulfaten, Monoglyceridsulfaten, Alkylsulfonaten, Alkylethersulfonaten, Alkylamidsulfonaten, Alkylarylsulfonaten, Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetaten, Alkyletherphosphaten, Acylsarcosinaten, Acylglutamaten, N-Acyltauraten und von Isethionaten, wobei der Alkyl- oder Acylrest aus einer Kohlenstoffkette mit 10 bis 20 Kohlenstoffatomen zusammengesetzt sind, oder daß das Co-Tensid auch aus polyoxalkylierten Alkylamiden oder Alkylethercarboxylsäuren ausgewählt ist.

14. Zusammensetzung gemäß Anspruch 12,
dadurch **gekennzeichnet**, daß
das nicht-ionische zusätzliche Co-Tensid aus polyeth- oder polypropoxylierten Alkoholen, α-Diolen, Alkylphenolen und Fettsäuren mit jeweils einer Fettkette von 8 bis 18 Kohlenstoffatomen, wobei die Anzahl der Ethylen- oder Propylenoxid-Gruppierungen 2 bis 50 und die Anzahl an Glyceryl-Gruppierungen 2 bis 30 betragen, aus Copolymeren von Ethylen- und Propylenoxiden, aus Kondensaten von Ethylen- und Propylenoxiden an Fettalkohole, aus polyethoxylierten Fettamiden, polyethoxylierten Fettaminen, oxethylierten Sorbitanfettsäureestern, Fettsäureestern von Zuckern, Fettsäureestern von Polyethylenglycolen, Fettestern von Glycolen und aus Aminoxiden ausgewählt ist.

15. Zusammensetzung gemäß Anspruch 12,
dadurch **gekennzeichnet**, daß
das amhotere zusätzliche Co-Tensid aus sekundären oder tertiären aliphatischen Aminderivaten, in denen der aliphatische Rest eine lineare oder verzweigte Kette mit 8 bis 18 Kohlenstoffatomen ist und mindetens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder C₈₋₂₀-Alkylamido-C₁₋₆-alkylsulfobetainen sowie aus Alkylpeptid- und Alkylimidazoliumbetainen ausgewählt ist.

16. Zusammensetzung gemäß Anspruch 13,
dadurch **gekennzeichnet**, daß
das kationische Co-Tensid aus quaternären Ammoniumsalzen ausgewählt ist.

17. Zusammensetzung gemäß einem der Ansprüche 1 bis 16,
dadurch **gekennzeichnet**, daß
das kosmetisch geeignete Medium aus Wasser oder aus einer Mischung aus Wasser und einem kosmetisch geeigneten Lösungsmittel zusammengesetzt ist.

18. Zusammensetzung gemäß einem der Ansprüche 1 bis 17,
dadurch **gekennzeichnet**, daß
sie in Form einer mehr oder weniger verdickten Flüssigkeit, eines Gels, einer Emulsion, einer hydroalkoholischen Lotion, einer Dispersion, eines Stück Seife oder eines Aerosol-Schaums vorliegt.

19. Zusammensetzung gemäß einem der Ansprüche 1 bis 18,
dadurch **gekennzeichnet**, daß
sie zusätzlich Additive enthält, die aus Schaumverstärkungsmitteln, Verdickungsmitteln, Sequestriermitteln, Elektrolyten, Parfüm-Produkten, Konservierungsmitteln, Fettalkoholen, mineralischen, pflanzlichen, tierischen oder synthetischen Ölen oder Wachsen, Ceramiden, UV-Filterstoffen, Abfangmitteln für freie Radikale, Perlmuttglanzmitteln, Bioziden, antibakteriellen Mitteln, Antischuppenmitteln, antiseborrheischen Mitteln, Mitteln gegen Parasiten, Abwehrmitteln, Farbstoffen, Pigmenten, Oxidiermitteln, Reduziermitteln, Hydratisiermitteln, anionischen, kationischen, nichtionischen oder amphoteren Polymeren, Vitaminen und aus α-Hydroxysäuren ausgewählt sind.

20. Verwendung der in jedem der Ansprüche 1 bis 19 definierten Zusammensetzung zur Behandlung und/oder zum Waschen keratinischer Materien, insbesondere der Haare und/oder der Haut.

21. Verfahren zum Waschen und/oder kosmetischen Londitionieren der Haare oder der Haut,
dadurch **gekennzeichnet**, daß
es darauf beruht, daß man auf die Haut oder die Haare eine wirksame Menge einer Zusammensetzung gemäß jedem der Ansprüche 1 bis 19 aufbringt, worauf nach dieser Aufbringung gegebenenfalls eine Spülung mit Wasser erfolgt.
